# EUROPEAN PATENT SPECIFICATION

(11) **EP 4 045 548 B1**
(45) Date of publication and mention of the grant of the patent: **23.10.2024**
(21) Application number: 20803697.0
(22) Date of filing: 14.10.2020
(51) Int. Cl.: C08F 220/18, C08K 5/00

(54) **COMPOSITION COMPRISING AN ALKALI-SWELLABLE COPOLYMER**
ZUSAMMENSETZUNG MIT EINEM ALKALI-QUELLBAREN COPOLYMER
COMPOSITION COMPRENANT UN COPOLYMÈRE POUVANT GONFLER DANS UN ALCALI

(30) Priority: 16.10.2019 US 201962915713 P; 16.10.2019 US 201962915715 P
(43) Date of publication of application: 24.08.2022
(73) Proprietor: Rohm and Haas Company, Collegeville, PA 19426 (US); Union Carbide Corporation, Seadrift, TX 77983 (US)
(72) Inventor: BUTTERICK, Robert, Collegeville, PA 19426 (US); CARDOEN, Gregoire, Collegeville, PA 19426 (US); CREAMER, Marianne, Collegeville, PA 19426 (US); EVEN, Ralph C., Blue Bell, PA 19422 (US); MAGNI, Kurt J., Oreland, PA 19075 (US); O'CONNOR, Ying, Collegeville, PA 19426 (US); WASSERMAN, Eric, Collegeville, PA 19426 (US)
(74) Representative: Houghton, Mark Phillip
(86) International application number: PCT/US2020/055452
(87) International publication number: WO 2021/076537

(56) References cited:
- EP-A1- 2 933 280
- WO-A2-2017/112584
- GB-A- 2 508 672
- US-A1- 2019 002 613

## Description

### CROSS-REFERENCE TO RELATED APPLICATIONS

This application claims the benefit of U.S. Provisional Patent Application Serial No. 62/915,715 and U.S. Provisional Patent Application Serial No. 62/915,713, both of which were filed October 16, 2019.

### BACKGROUND

In developing compositions that may have long shelf-lives, achieving the desired viscosity profiles can be challenging. For example, in certain cleaning compositions, it can be desirable to have a composition that maintains a homogeneous distribution during long shelf-lives that also exhibits a sufficient shear thinning effect to allow the composition to be dispensed from its packaging. The property of having much higher viscosity at low shear than at high shear, known as "shear thinning," can be imparted by many polymers when dissolved, including those in the hydrophobe-modified alkali-swellable emulsion family of reticulated vinyl polymers (HASEs).

EP 2 933 280 A1, GB 2 508 672 A, US 2019/002613 A1 and WO 2017/112584 A2 relate to alkali-swellable or thickening compositions based on different acrylic copolymers.

While several hydrophobe-modified alkali-swellable emulsion polymers have been developed, further improvements are needed.

### BRIEF SUMMARY

Disclosed herein according to the appended claims is a composition comprising an alkali-swellable copolymer.

In an aspect, a composition comprises an alkali-swellable copolymer. The alkali-swellable copolymer is derived from a mixture comprising 15 to 50 weight percent (wt%) based on the total weight of the monomers in the mixture of an ethylenically unsaturated carboxylic acid; 30 to 70 wt% based on the total weight of the monomers in the mixture of an ethylenically unsaturated C₁₋₂ alkyl carboxylate ester; and 15 to 40 wt% based on the total weight of the monomers in the mixture of a nonionic ethylenically unsaturated surfactant monomer of the Formula (A) described below; and less than or equal to 0.15 wt% based on the total weight of the monomers in the mixture of a multifunctional monomer contains greater than 1 vinyl group. The composition has a pH of greater than or equal to 6. The composition can optionally comprise a surfactant.

In another aspect, a composition comprises 0.1 to 10 wt% of an alkali-swellable copolymer based on the total weight of the composition and 5 to 50 wt% of at least one of a nonionic surfactant or an anionic surfactant based on the total weight of the composition. The alkali-swellable copolymer is derived from a mixture comprising 15 to 50 wt%, or 20 to 35 wt% based on the total weight of the monomers in the mixture of methacrylic acid and an optional acrylic acid, wherein the optional acrylic acid is present in an amount of less than or equal to 50 wt% based on the total weight of the methacrylic acid and the optional acrylic acid; 30 to 70 wt%, or 35 to 55 wt% based on the total weight of the monomers in the mixture of ethyl (meth)acrylate; and 15 to 40 wt%, or 15 to 35 wt% based on the total weight of the monomers in the mixture of a nonionic ethylenically unsaturated surfactant monomer of the Formula (A) described below; 0.01 to 0.15 wt% based on the total weight of the monomers in the mixture of a multifunctional monomer containing greater than 1 vinyl group. The alkali-swellable copolymer is the product of an aqueous emulsion polymerization. The composition has a pH of greater than or equal to 6.

The above described and other features are exemplified by the following detailed description and claims.

### DETAILED DESCRIPTION

It is often desired that a composition contains a dispersion of insoluble components. These insoluble components can perform a function such as removing soils, or they can provide an aesthetic benefit by reflecting light or carrying perfume. In the case of personal care compositions, the composition often can contain insoluble components that confer a tactile or cosmetic benefit, for example, to the skin, nails, or hair. In order to ensure a relatively homogeneous distribution of the insoluble component in the composition, the composition should have a high viscosity of greater than or equal to 20 pascal seconds (Pa•s) at low shear rates of 0.01 inverse seconds (s⁻¹) so that the force of gravity or buoyancy acting on the insoluble component is resisted on timescales relevant to the shelf-life of the product (for example, a shelf-life of one year). Moreover, the composition should be capable of being quickly dispensed from its container by gravity, where the viscosity at shear rates typical for pouring from bottles at 10 to 100 s⁻¹ should be relatively low, for example, less than or equal to 10 Pa•s.

An alkali-swellable copolymer containing a relatively low level of polyfunctional (crosslinking) monomer was discovered that is capable of achieving the desired viscosity profile having a strongly shear-thinning rheology and a high low-shear viscosity value associated with suspension of the insoluble component.

The alkali-swellable copolymer is derived from a mixture comprising 15 to 50 wt%, or 20 to 50 wt% based on the total weight of the monomers in the mixture of an ethylenically unsaturated carboxylic acid; 30 to 70 wt% based on the total weight of the monomers in the mixture of an ethylenically unsaturated C₁₋₂ alkyl carboxylate ester; and 15 to 40 wt% based on the total weight of the monomers in the mixture of a nonionic ethylenically unsaturated surfactant monomer of the Formula (A); and less than or equal to 0.15 wt% based on the total weight of the monomers in the mixture of a multifunctional monomer containing greater than 1 vinyl group. In other words, the alkali-swellable copolymer is polymerized from the monomers present in the mixture and comprises residues of the ethylenically unsaturated carboxylic acid, the ethylenically unsaturated C₁₋₂ alkyl carboxylate ester, the nonionic ethylenically unsaturated surfactant monomer of the formula (A), and the multifunctional monomer in the respective amounts.

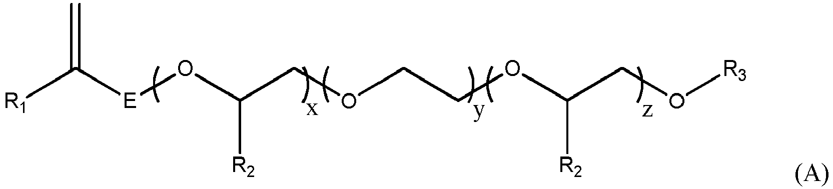

The alkali-swellable copolymer can be considered a HASE copolymer. The composition can have a pH of greater than or equal to 6.

In Formula (A), R₁ is a methyl group or hydrogen, each R₂ independently is a methyl group or an ethyl group, R₃ is a linear C₈₋₁₄ alkyl group, x is an integer 0 to 3, y is an integer 10 to 40, z is an integer 0 to 5, and E is a difunctional group that can be -(CH₂)-, -(CH₂CH₂)-, or -(C=O)-. E can be a difunctional group that can is selected from the group consisting of -(CH₂)-, -(CH₂CH₂)-, or -(C=O)-. R₃ can be a linear C₁₂₋₁₄ alkyl group.

The alkali-swellable copolymer is derived from a mixture comprising the ethylenically unsaturated carboxylic acid, the ethylenically unsaturated C₁₋₂ alkyl carboxylate ester, the nonionic ethylenically unsaturated surfactant monomer of the Formula (A), and the multifunctional monomer containing greater than 1 vinyl group.

The ethylenically unsaturated carboxylic acid can comprise at least one of acrylic acid or methacrylic acid. The ethylenically unsaturated carboxylic acid can comprise methacrylic acid. The ethylenically unsaturated carboxylic acid can comprise acrylic acid and methacrylic acid in a weight ratio of 1:1 to 1:4, or 1:1.5 to 1:3. The ethylenically unsaturated carboxylic acid can comprise less than or equal to 50 wt% of acrylic acid based on the total weight of the ethylenically unsaturated carboxylic acid.

The ethylenically unsaturated C₁₋₂ alkyl carboxylate ester can comprise at least one of ethyl acrylate, ethyl methacrylate, methyl acrylate, or methyl methacrylate. It was found that limiting the alkyl group to a C₁₋₂ alkyl group in the ethylenically unsaturated C₁₋₂ alkyl carboxylate ester resulted in a composition with an improved viscosity. For example, replacing the C₁₋₂ alkyl with a C₄ alkyl group of a butyl acrylate, can result in a significant decrease in the peak viscosity value.

The nonionic ethylenically unsaturated surfactant monomer of the Formula (A) comprises a vinyl containing endgroup, an ethylene oxide center portion, and a lipophilic endgroup. The vinyl containing endgroup is reactive to allow polymerization with the ethylenically unsaturated carboxylic acid and the ethylenically unsaturated C₁₋₂ alkyl carboxylate ester. The vinyl containing endgroup is connected to the ethylene oxide center portion via a linker E that is a difunctional group. The difunctional group can be -(CH₂)-, -(CH₂CH₂)-, or -(C=O)-.

The ethylene oxide center portion provides hydrophilicity to the alkali-swellable copolymer. The chain lengths of the respective ethylene oxide center portions can be 10 to 48, or 10 to 40, or 10 to 30 repeat units and can optionally include methyl or ethyl pendent groups. In Formula (A), x can be 0 to 3, or 1 to 3; y can be 10 to 40, or 10 to 30, or 20 to 30; and z can be 0 to 5, or 1 to 4. The respective ethylene oxide center portions in the nonionic ethylenically unsaturated surfactant monomer of the Formula (A) can vary, for example, such that at least 50%, or 75 to 100% of the ethylene oxide center portions based on the total number of ethylene oxide center portions have a chain length of 10 to 48 repeat units. It is noted that the ethylene oxide center portion can be blocky, for example, as illustrated in Formula (A) or can be random.

The lipophilic endgroup can be linear C₈₋₁₄ alkyl group, or a linear C₁₂₋₁₄ alkyl group derived from a linear C₈₋₁₄ alkyl alcohol or a linear C₁₂₋₁₄ alkyl alcohol. The lipophilic endgroups can be of synthetic or natural origin and can contain a range of different chain lengths. It is therefore understood that the C₈₋₁₄ alkyl chain length can be the predominant chain length of the lipophilic endgroups present in the alkali-swellable copolymer in an amount of at least 50%, or 75 to 100% based on the total number of lipophilic endgroups.

The nonionic ethylenically unsaturated surfactant monomer can comprise a methacrylate ester of a C₁₂₋₁₄ linear alcohol ethoxylate.

The multifunctional monomer can be used to crosslink the alkali-swellable copolymer or to copolymerize the alkali-swellable copolymer with a different component comprising a pendant reactive group. The multifunctional monomer can comprise two or more reactive groups, for example, two or more vinyl groups. The multifunctional monomer can comprise 2 to 6, or 2 to 4, or 2 to 3 vinyl groups. The multifunctional monomer can comprise at least one of di(meth)acrylate of ethylene glycol, di(meth)acrylate of polyethylene glycol, di(meth)acrylate of triethylene glycol, di(meth)acrylate of 1,3-butylene glycol, 1,6-butylene glycol di(meth)acrylate, 1,6-hexanediol di(meth)acrylate, neopentyl glycol di(meth)acrylate, 1,9-nonanediol di(meth)acrylate tri(meth)acrylate of trimethylolpropane, tri(meth)acrylate trimethylolethane, tri(meth)acrylate pentaerythritol, tri(meth)acrylate of tetramethylolmethane, ditrimethylolpropane tetra(meth)acrylate, tetramethylolmethane tetra(meth)acrylate, pentaerythritol tetra(meth)acrylate, dipentaerythritol hexa(meth)acrylate, penta(meth)acrylate of dipentaerythritol, allyl (meth)acrylate, diallylphthalate, diallyl itaconate, diallyl fumarate, diallyl maleate, polyallyl ethers of sucrose with 2 to 8 groups per molecule, pentaerythritol diallyl ether, pentaerythritol triallyl ether, pentaerythritol tetraallyl ether, trimethylolpropane diallyl ether, trimethylolpropane triallyl ether, divinyl glycol, divinyl benzene, divinylcyclohexyl, or methylenebisacrylamide. The multifunctional monomers can comprise at least one of trimethylolpropane triacrylate, trimethylolpropane diallyl ether, divinyl benzene, allyl methacrylate, diacrylobutylene, ethylene glycol dimethacrylate, or diallyl phthalate. The multifunctional monomer can comprise at least one of trimethylolpropane triacrylate, trimethylolpropane diallyl ether, or diallyl phthalate.

The mixture can further comprise a hydrophilic comonomer, for example, having at least one of a hydroxyl, a carboxylic acid, or a sulfonic acid group; or at least one of the hydroxyl or the carboxylic acid group. The hydrophilic comonomer can comprise at least one of 2-hydroxyethyl (meth)acrylate (HEMA or HEA), itaconic acid, or acrylamido-2-methylpropanesulfonic acid. The hydrophilic comonomer can comprise at least one of 2-hydroxyethyl (meth)acrylate (HEMA or HEA) or itaconic acid.

The alkali-swellable copolymer can be free of, for example, comprising 0 to 0.5 wt%, or 0 wt%, a residue derived from a sulfonate monomer based on the total weight of the monomers. The alkali-swellable copolymer can be free of, for example, comprising 0 to 0.5 wt%, or 0 wt%, a residue derived from an ethylenically unsaturated C₃₋₄ alkyl carboxylate ester based on the total weight of the monomers.

The alkali-swellable copolymer can be prepared using known polymerization techniques, for example, via aqueous or inverse emulsification procedures, or precipitation or solution polymerization processes. The alkali-swellable copolymer can be prepared using an aqueous emulsion polymerization at a pH of greater than or equal to 6, where such a polymerization can result in an alkali-swellable copolymer that can be free of a core-shell structure. The polymerization reaction can be catalyzed via free-radical initiator (for example, a peroxygen compound) and an optional chain transfer agent. The free-radical initiator can comprise at least one of a peroxide, a hydroperoxide, a persulfate, an organic peroxide, or an initiator such as azobisisobutyronitrile. The free-radical initiator can be present in an amount of 0.01 to 3 wt% based on the total weight of the monomers. The chain transfer agent can comprise a compound having a mercapto group (for example, a long chain alkyl mercaptan or a thioester (for example, dodecyl-, octyl-, tetradecyl- or hexadecyl-mercaptan or butyl-, isooctyl- or dodecyl-thioglycolate)). The chain transfer agent can be present in an amount of 0.01 to 5 wt%, or 0.1 to 1 wt% based on the total weight of the monomers.

Alternatively, the alkali-swellable copolymer can be polymerized using known polymerization techniques, wherein the reactant monomers and initiator are dissolved in an appropriate solvent, for example, at least one of toluene, xylene, or tetrahydrofuran. Polymerization can be accomplished within the time necessary at a given reaction temperature, for example, 60 to 80 degrees Celsius (°C) for 2 to 24 hours. The alkali-swellable copolymer can be isolated through normal separation techniques, including solvent stripping.

The polymerization temperature can be 60 to 90 °C. The alkali-swellable copolymer can be recovered by filtration and the copolymer can be dried. Examples of polymerization methods can be found in U.S. Pat. Nos. 4,384,096, 4,663,385, 4,429,097 and 4,514,552. The weight average molecular weight of uncrosslinked alkali-swellable copolymer can be 100,000 to 1 million grams per mole based on polystyrene standards.

The composition can comprise 0.1 to 10 wt% of the alkali-swellable copolymer based on the total weight of the composition.

The composition can comprise a surfactant. The surfactant can be present from the emulsion polymerization and can comprise at least one of an anionic surfactant or a nonionic surfactant. An additional surfactant can be present in the composition, for example, at least one of a cationic surfactant, an amphoteric surfactant (for example, comprising both anionic and cationic groups), or a zwitterionic surfactant provided they are compatible with the composition. The total amount of surfactant that can be present can be greater than 0 to 50 wt%, or 5 to 50 wt%, or 15 to 50 wt%, or 15 to 35 wt% based on the total weight of the composition. Examples of surfactants include C₈₋₁₈ fatty acids or their water soluble salts, water soluble sulfates of C₈₋₁₈ alcohols, sulfonated alkylaryl compounds (for example, dodecylbenzene sulfonate), alkylphenoxy polyethoxyethanols (for example, with C₇₋₁₈ alkyl groups and 9 to 40 oxyethylene units), alkylene oxide derivatives of long chain carboxylic acids (for example, of lauric, myristic, palmitic, or oleic acids), alkylene oxide derivatives of long chain alcohols (for example, of lauryl or cetyl alcohols), alkanolamides, or polyglucosides (for example, the alkyl polyglucosides). The alkylene oxide of the surfactant can comprise at ethylene oxide and/or propylene oxide units.

The surfactant can comprise at least one of an anionic surfactant or a nonionic surfactant. The surfactant can comprise the anionic surfactant. The anionic surfactant can comprise at least one of a carboxylate, sulfonate, sulfate, or phosphate solubilizing group. The anionic surfactant can comprise at least one of a C₉₋₁₃ alkyl benzene sulfonate, a C₁₂₋₁₅ alkane sulfonate, a C₁₀₋₁₈ isethionate, a C₁₀₋₁₈ glutamate, or a C₁₀₋₁₈ glycinate. The anionic surfactant can comprise at least one of sodium dodecylbenzenesulfonate, sodium laureth sulfate, sodium lauryl sulfate, sodium tridecyl ether sulfate, a dioctyl sulfosuccinate sodium salt, or a sodium salt of an alkylaryl polyether sulfonate.

The surfactant can comprise the nonionic surfactant. The nonionic surfactant can comprise at least one of an amide group, a hydroxyl group, or alkylene oxide chains. The nonionic surfactant can comprise at least one of a linear or branched C₈₋₃₀ fatty alcohol ethoxylates (for example, capryl alcohol ethoxylate, lauryl alcohol ethoxylate, myristyl alcohol ethoxylate, cetyl alcohol ethoxylate, stearyl alcohol ethoxylate, cetearyl alcohol ethoxylate, sterol ethoxylate, oleyl alcohol ethoxylate, or behenyl alcohol ethoxylate), an alkylphenol alkoxylate (for example, octylphenol ethoxylate), an alkylpolyglucoside, or an ethylene oxide-propylene oxide copolymer. The nonionic surfactant can comprise at least one of lauryl alcohol ethoxylate, an alkylaryl polyether alcohol, an ethylene oxide-propylene oxide copolymer, or an alkylpolyglucoside.

The anionic surfactant can be present in an amount of greater than 0 to 50 wt%, or 0.5 to 30 wt% based on the total weight of the composition. The nonionic surfactant can be present in an amount of greater than 0 to 50 wt%, or 0.5 to 15 wt% based on the total weight of the composition. The composition can comprise both the anionic surfactant and the nonionic surfactant in a weight ratio of 2.5:1 to 1.5:1.

The surfactant can comprise the cationic surfactant, for example, comprising at least one of an amine or an ammonium solubilizing group. The cationic surfactant can comprise at least one of lauryl pyridinium chloride, octylbenzyltrimethyl-ammonium chloride, dodecyl trimethylammonium chloride, or an alkylene oxide condensate of a primary fatty acid amine. Conversely, the surfactant can be free of a cationic surfactant, for example, comprising 0 wt% of the cationic surfactant based on the total weight of the surfactant.

The surfactant can comprise the amphoteric surfactant, for example, comprising at least one of an alkali metal, an alkaline earth metal, an ammonium or substituted ammonium salt of an alkyl amphodipropionate, an alkyl amphoacetate, an alkyl amphodiacetate, an alkyl amphoglycinate, an alkyl amphopropionate, an alkyl iminopropionate, an alkyl iminodipropionate, or an alkyl amphopropylsulfonate. The amphoteric surfactant can comprise at least one of cocoamphoacetate, cocoamphopropionate, cocoamphodiacetate, lauroamphoacetate, lauroamphodiacetate, lauroamphodipropionate, lauroamphodiacetate, cocoamphopropylsulfonate, caproamphodiacetate, caproamphoacetate, caproamphodipropionate, stearoanphoacetate, cocoamidopropyl hydroxysultaine, sodium lauroamphoacetate, sodium lauroamphopropionate, disodium lauroamphodiacetate, sodium cocoamphoacetate, or disodium cocoamphodiacetate. Conversely, the surfactant can be free of the amphoteric surfactant, for example, comprising 0 wt% of the amphoteric surfactant based on the total weight of the surfactant.

The surfactant can comprise the zwitterionic surfactant. The zwitterionic surfactant can comprise at least one of a betaine surfactant or a sultaine surfactant. The zwitterionic surfactant can comprise at least one of decyl dimethyl betaine, undecyl dimethyl betaine, dodecyl dimethyl betaine, tridecyl dimethyl betaine, tetradecyl dimethyl betaine, coco dimethyl betaine, hexadecyl dimethyl betaine, heptadecyl dimethyl betaine, octadecyl dimethyl betaine, dodecylamidopropyl dimethyl betaine, cocoamidopropyl betaine cocoamidopropyl dimethyl betaine, oleylamidopropyl betaine, lauryl dihydroxypropyl glycinate, lauryl di(hydroxy-poly(ethoxy)) glycinate, lauryl bis-(2-hydroxy-ethyl)carboxy methyl betaine, stearyl bis-(2-bydroxy-propyl)carboxymethyl betaine, cocodimethyl sulfopropyl betaine, stearyldimethyl sulfopropyl betaine, lauryl dimethyl sulfoethyl betaine, lauryl bis-2-hydroxy-ethyl)sulfopropyl betaine, or cocoamidopropyl hydroxysultaine.

The composition can comprise an insoluble component. The insoluble component can perform a function that can provide the aesthetic benefits by reflecting light or carrying a perfume/active. The insoluble component can function in the personal care composition as an opacifying material, a pearlescent material, a cosmetic agent (for example, a vitamin for nourishing skin or hair), or an exfoliating agent. In the case of personal care compositions, the composition can contain insoluble components that confer a tactile or cosmetic benefit to the skin or hair. The insoluble component can be derived from at least one of an inorganic, an organic, a natural, or a synthetic source. The insoluble component can comprise at least one of a silicone, a gaseous bubble, an anti-dandruff agent (for example, zinc pyrithione or 2-hydroxypuridine-1-oxide), almond meal, apricot seed powder, barley flour, corn cob meal, corn cob powder, corn flour, corn meal, corn starch, oat bran, oat flour, oatmeal, peach pit powder, pecan shell powder, jojoba seed powder, pumice, rice bran, rye flour, soy flour, walnut shell powder, wheat bran, wheat flour, wheat starch, lufa, clay, Fuller's earth, alumina, aluminum oxide, aluminum silicate, palygorskite, bismuth oxychloride, boron nitride, calcium carbonate, calcium phosphate, calcium pyrophosphate, calcium sulfate, cellulose chalk, chitin, diatomaceous earth, dicalcium phosphate, dicalcium phosphate dihydrate, hydrated silica, hydroxyapatite, kaolin, iron oxide, magnesium trisilicate, tin oxide, mica, titanium dioxide, titanium dioxide coated mica, tricalcium phosphate zirconium silicate, microcrystalline cellulose, montmorillonite, polybutylene, polyethylene, polyisobutylene, polymethylstyrene, polypropylene, polystyrene, polyurethane, nylon, polytetrafluoroethylene, polyhalogenated olefins, hydrogenation products of jojoba oil, interesterification products of jojoba oil, sericite, silica, silk, sodium bicarbonate, sodium silicoaluminate, synthetic hectorite, talc, a wax (for example, paraffin, carnauba, ozocerite, candelilla), or a resin (for example, urea-formaldehyde). The insoluble component can comprise at least one of a clay (for example, a swellable clay), a liposome, a particulate sponge, a cosmetic bead, or an encapsulant (for example, that is designed to burst upon use). The shape of the insoluble component in composition is not limited and can be in the form of a sphere, an ovoid, a flake, an agglomerate, an irregular shape, etc. The composition can be a cleaning composition and can include an insoluble component. The composition can be a personal care composition and can include an insoluble component.

The composition can comprise an additive. The additive can comprise at least one of an enzyme, a dispersant polymer, a conditioning polymer, a solvent (for example, water or an alkyl alcohol), a hydrotropic electrolyte, an oxygen bleach, a bleach activator, an inorganic builder, an organic builder, a foam inhibitor, an antimicrobial agent, a germicide, a fungicide, an antioxidant, an antistatic agent, an ironing aid, a UV light absorber, a fatty acid, a fatty acid salt, a silicon fluid, an optical brightener, a dye transfer inhibitor, a dye, a fragrance, a salt, or a preservative.

The composition can comprise an alkali-swellable copolymer. The alkali-swellable copolymer can be derived from a mixture comprising: 15 to 50 wt% based on the total weight of the monomers in the mixture of an ethylenically unsaturated carboxylic acid; 30 to 70 wt% based on the total weight of the monomers in the mixture of an ethylenically unsaturated C₁₋₂ alkyl carboxylate ester; 15 to 40 wt% based on the total weight of the monomers in the mixture of a nonionic ethylenically unsaturated surfactant monomer of the Formula (A); and less than or equal to 0.15 wt% based on the total weight of the monomers in the mixture of a multifunctional monomer containing greater than 1 vinyl group. The composition can have a pH of greater than or equal to 6. The composition can comprise 0.1 to 10 wt% of the alkali-swellable copolymer. The ethylenically unsaturated carboxylic acid can comprise at least one of acrylic acid or methacrylic acid. The ethylenically unsaturated C₁₋₂ alkyl carboxylate ester can comprise at least one of ethyl acrylate, ethyl methacrylate, methyl acrylate, or methyl methacrylate. The nonionic ethylenically unsaturated surfactant monomer can comprise a methacrylate ester of a C₁₂₋₁₄ linear alcohol ethoxylate. The multifunctional monomer can comprise at least one of trimethylolpropane triacrylate, trimethylolpropane diallyl ether, or diallyl phthalate. The alkali-swellable copolymer can be free of a residue derived from a sulfonate monomer. The composition can comprise a surfactant. The composition can comprise greater than 0 to 50 wt% based on the total weight of the composition of the surfactant. The surfactant can comprise an anionic surfactant. The anionic surfactant can comprise at least on of sodium dodecylbenzenesulfonate, sodium laureth sulfate, sodium lauryl sulfate, sodium tridecyl ether sulfate, a dioctyl sulfosuccinate sodium salt, or a sodium salt of an alkylaryl polyether sulfonate. The composition can comprise an anionic surfactant can be present in an amount of 0.5 to 30 wt% based on the total weight of the composition. The surfactant can comprise a nonionic surfactant. The nonionic surfactant can comprise at least one of lauryl alcohol ethoxylate, an alkylaryl polyether alcohol, an alkylpolyglucoside, or an ethylene oxide-propylene oxide copolymer. The nonionic surfactant can be present in an amount of 0.5 to 15 wt% based on the total weight of the composition. The surfactant can comprise a zwitterionic surfactant. The zwitterionic surfactant can comprise at least one of dimethyldodecylamine N-oxide or cocamidopropyl betaine. The zwitterionic surfactant can be present in an amount of 0.5 to 15 wt% based on the total weight of the composition. The alkali-swellable copolymer can be the product of an aqueous emulsion polymerization. The composition can be a cleaning composition, for example, a liquid laundry detergent or a liquid dishwashing detergent. The composition can be a personal care cleanser. The composition can be a leave-on personal care product. The composition can be a personal care product and can comprise a zwitterionic surfactant. The composition can further comprise at least one of an enzyme, a dispersant polymer, a conditioning polymer, a solvent, a hydrotropic electrolyte, an oxygen bleach, a bleach activator, an inorganic builder, an organic builder, a fatty acid, a fatty acid salt, a silicon fluid, an optical brightener, a dye transfer inhibitor, a dye, a fragrance, a salt, or a preservative. The composition can further comprise an insoluble component.

In a specific example, the composition can comprise 0.1 to 10 wt% of an alkali-swellable copolymer based on the total weight of the composition, wherein the alkali-swellable copolymer is derived from a mixture comprising 15 to 50 wt%, or 20 to 35 wt% based on the total weight of the monomers in the mixture of methacrylic acid and an optional acrylic acid, wherein the optional acrylic acid is present in an amount of less than or equal to 50 wt% based on the total weight of the methacrylic acid and the optional acrylic acid; 30 to 70 wt%, or 35 to 55 wt% based on the total weight of the monomers in the mixture of ethyl (meth)acrylate; 15 to 40 wt%, or 15 to 35 wt% based on the total weight of the monomers in the mixture of a nonionic ethylenically unsaturated surfactant monomer of the Formula (A); wherein R₁ is a methyl group or hydrogen, each R₂ independently is a methyl group or an ethyl group, R₃ is a linear C₁₂₋₁₄ alkyl group, x is an integer 0 to 3, y is an integer 10 to 40, z is an integer 0 to 5, and E is -(CH₂)-, -(CH₂CH₂)-, or -(C=O)-; and 0.01 to 0.15 wt% based on the total weight of the monomers in the mixture of a multifunctional monomer containing greater than 1 vinyl group. The composition can comprise 5 to 50 wt% of at least one of a nonionic surfactant or an anionic surfactant based on the total weight of the composition, the alkali-swellable copolymer can be the product of an aqueous emulsion polymerization; and the composition can have a pH of greater than or equal to 6.

The method of forming the composition is not particularly limited. An example of forming the composition comprises mixing the alkali-swellable copolymer with deionized water to form a dispersion. The pH of the dispersion can be adjusted during formation of the dispersion (for example, by adding a first amount of deionizing water, adjusting the pH and adding a second amount of deionizing water) or after formation of the dispersion. Remaining components such as the surfactant or the additive can be added before or after adjusting the pH. Forming the dispersion prior to forming the composition can allow of easier and more accurate adjustment of the viscosity.

The composition can be thickened by adjusting the pH. For example, the pH can be adjusted to greater than or equal to 6, or greater than or equal to 7; or 6 to 13. The pH can be adjusted using a neutralizing agent. The neutralizing agent can comprise a base. The base can comprise least one of an amine base, an alkali metal, or ammonium hydroxide. The base can comprise at least one of sodium hydroxide, ammonium hydroxide, or triethanolamine (TEA). Alternatively, the alkali-swellable copolymer can first be neutralized in aqueous dispersion and then mixed to form the composition.

The composition can exhibit a non-Newtonian shear thinning viscosity, where the viscosity decreases in a certain range with increasing shear stress. The composition can have a viscosity of greater than or equal to 20 Pa•s at a shear rate of 0.01 s⁻¹. The composition can have a viscosity of greater than or equal to 1 Pa•s at a shear rate of 30 s⁻¹. The composition can have a peak viscosity of greater than or equal to 20 Pa•s, or 20 to 350 Pa•s, 25 to 320 Pa•s. The viscosity can be determined using a lower temperature-controlled stainless-steel Peltier plate and an upper 40 millimeter stainless-steel 2 degree (°) cone according to the following procedure: conditioning at 25 °C for 10 seconds, pre-shearing at 21 s⁻¹ for 10 seconds, and equilibrating for 300 seconds, oscillating at 25 °C at a constant strain of 1.0 percent, and a logarithmic frequency sweep of 100 to 0.01 radians per second (rad/s) with 10 points per decade, repeating the conditioning step, and performing the flow step. The flow step included a logarithmic stress sweep at 0.1 to 400 Pascal (Pa) with 10 points per decade at a temperature of 25 °C, an equilibration time of 20 seconds, an averaging time of 30 seconds, and a scaled time average scale factor of 1.0.

The composition can be stable with the insoluble component(s) remaining suspended in the composition for greater than or equal to 6 months, or greater than or equal to 1 year at a temperature of 23 °C.

The composition can be a personal care product, for example, a cosmetic, shampoo, conditioner, two-in-one shampoo conditioners, a hair colorant, a hair relaxer, a hair smoothing product, a leave-on hair product (for example, a conditioner, a serum, a spray, or a gel), a hair styling product, body wash, facial wash, hand soap, soap, toner, serum, lotion, cream, a shaving product, a wipe, a skin treatment (for example, an anti-acne product or an anti-aging protectant), a sunblock, a skin color product, a foundation, a primer, a makeup remover, a bath product (for example, a bath oil or a bubble bath), a nail care product (for example, a polish, a polish remover, a hardener, a cuticle remover, or a softener), an oral care product (for example, a toothpaste or a mouth wash), an air freshener. The personal care product can comprise a surfactant, for example, a zwitterionic surfactant. As used herein, the term personal care product can refer to products for use on living beings.

The composition can be cleaning composition, for example, a laundry detergent, a dishwashing detergent, a surface cleaner (for example, for counter tops, floors, sinks, or toilet bowls). The cleaning composition can be used for cleaning textiles or non-textile surfaces (for example, that comprise at least one of metal, paint, wood, plastic, ceramic, or tile). The cleaning composition can be a liquid, a foam, a gel, a spray, an emulsion (for example, oil-in-water or water-in-oil), an ointment, or a paste. As used herein, the term cleaning composition can refer to compositions used for cleaning non-living things.

The following examples are provided to illustrate the present disclosure. The examples are merely illustrative and are not intended to limit devices made in accordance with the disclosure to the materials, conditions, or process parameters set forth therein.

### Examples

The components used in the examples are listed in Table 1.

| Table 1 | |
|---|---|
| Acronym | Component |
| AA | Acrylic acid, monomer |
| BA | Butyl acrylate, monomer |
| EA | Ethyl acrylate, monomer |
| EO | Ethylene oxide |
| MAA | Methacrylic acid, monomer |
| AE-1 | Lauryl/myristyl ethoxylate-23, methacrylate ester, alcohol ethoxylate |
| AE-2 | Cetyl/stearyl ethoxylate-20, methacrylate ester, alcohol ethoxylate |
| AE-3 | Behenyl ethoxylate, methacrylate ester, alcohol ethoxylate |
| AE-4 | BIO-SOFT^{™} N25-7, linear alcohol (C₁₂₋₁₅) ethoxylate, 7 mol EO, from Stepan Co. |
| PM-1 | Trimethylolpropane triacrylate (TMPTA) |
| PM-2 | Trimethylolpropane diallyl ether (TMPDAE) |
| PM-3 | Diallyl phthalate (DAP) |
| SDBS | NACCONOL^{™} 90G, sodium dodecylbenzenesulfonate, surfactant, from Stepan Co. |
| SLES | STEOL^{™} CS-460, sodium laureth sulfate, 3 mol EO, surfactant, from Stepan Co. |

### Example 1: Preparation of alkali-swellable copolymers

Alkali-swellable copolymers 1-16 were prepared by copolymerizing the monomers in the amounts shown in Table 2 using known emulsion polymerization methods.

### Example 2: Preparation of a surfactant mixture

A surfactant concentrate was prepared according to the amounts specified in Table 3. The surfactant concentrate was prepared at ambient temperature except where specified and the actual added amounts of each ingredient were recorded and used to calculate the amount of water added in the final step.

| Table 3 | | | |
|---|---|---|---|
| | % active in raw material | amount added, g | active concentration in concentrate, % |
| DI water | 100 | 415.56 | |
| SDBS | 90 | 88.89 | 12.7 |
| SLES | 60 | 33.33 | 3.2 |
| Propylene glycol | 100 | 35.00 | 5.6 |
| Ethanol | 100 | 15.00 | 2.4 |
| AE-4 | 100 | 40.00 | 6.4 |

The surfactant concentrate was prepared by charging a beaker with deionized (DI) water while stirring. SDBS was added gradually with rapid stirring at 300 to 500 revolutions per minute (rpm) to avoid the formation of lumps. Propylene glycol and ethanol were added and the mixture stirred for 5 minutes. SLES was melted by heating to 30 °C and then added gradually to the mixture and the mixture stirred for 5 minutes. Finally, AE-4 was added to the mixture and the mixture stirred for 5 minutes.

### Example 3: Preparation of compositions

An amount of the respective polymer of polymers 1-16 was weighed and mixed with an initial amount of deionized water to ensure smooth dispersion of the concentrate. While mixing with an overhead stirrer at 500 revolutions per minute, an amount of concentrate was slowly poured into the jar. The pH was adjusted to 7.0 using sodium hydroxide (5 volume percent in deionized water). Next, an amount of deionized water was added to reach the intended final concentration, and the mixture stirred for an additional hour. The amounts used are shown in Table 4 in grams (g) and the weight percent (wt%) of the active ingredient in the composition indicated.

| Table 4 | | | |
|---|---|---|---|
| | % active in raw material | amount added (g) | active concentration in concentrate (wt%) |
| Polymer 1-16 | 31 | 4.0 | 1.25 |
| Water, first addition | 100 | 20.9 | |
| Concentrate | | 62.8 | |
| *SDBS* | 12.7 | | 8.00 |
| *SLES* | 3.2 | | 2.00 |
| *propylene glycol* | 5.6 | | 3.50 |
| *ethanol* | 2.4 | | 1.50 |
| *AE-4* | 6.4 | | 4.00 |
| sodium hydroxide (5 vol%) | | 2.4 | |
| Water, second addition | | 9.9 | |
| Total | | 100 | |

### Example 4: Rheological Analysis of the polymers of Example 1

Prior to the rheological analysis, the compositions containing the respective polymers were centrifuged at 5,000 revolutions per minute for 20 minutes to remove bubbles.

After centrifugation, the samples were allowed to rest for at least 16 hours prior to rheological testing. The samples were analyzed on a DHR-3 rheometer (TA Instruments), with an initial frequency sweep at constant strain followed by a flow rheology test at increasing shear rate.

| Table 2 | | | | | | | | | | | | | | | | |
|---|---|---|---|---|---|---|---|---|---|---|---|---|---|---|---|---|
| Polymer | 1 | 2 | 3 | 4 | 5 | 6 | 7 | 8 | 9 | 10 | 11 | 12 | 13 | 14 | 15 | 16 |
| EA (wt%) | 48.1 | 46.1 | 52 | 48.1 | 48.1 | 46.1 | 43 | 37 | 48.1 | 48.1 | 62 | 57 | 48.1 | - | 48.1 | 48.1 |
| BA (wt%) | - | - | - | - | - | - | - | - | - | - | - | - | - | 48.1 | - | - |
| AE | AE-1 | AE-1 | AE-1 | AE-1 | AE-1 | AE-1 | AE-1 | AE-1 | AE-1 | AE-1 | AE-1 | AE-1 | AE-1 | AE-1 | AE-2 | AE-3 |
| AE (wt%) | 20 | 22 | 15 | 20 | 20 | 22 | 20 | 30 | 20 | 20 | 5 | 10 | 20 | 20 | 20 | 20 |
| MAA (wt%) | 31.9 | 21.9 | 33 | 31.9 | 31.9 | 21.9 | 37 | 33 | 31.9 | 31.9 | 33 | 33 | 31.9 | 31.9 | 31.9 | 31.9 |
| AA (wt%) | - | 10 | - | - | - | 10 | - | - | - | - | - | - | - | - | - | - |
| PM | - | - | PM-1 | PM-1 | PM-1 | PM-1 | PM-1 | PM-1 | PM-2 | PM-3 | PM-1 | PM-1 | PM-1 | PM-1 | PM-1 | PM-1 |
| PM (wt%) | - | - | 0.035 | 0.035 | 0.035 | 0.035 | 0.035 | 0.035 | 0.105 | 0.105 | 0.035 | 0.035 | 0.35 | 0.035 | 0.035 | 0.035 |

| Rheology of the Compositions | | | | | | | | | | | | | | | | |
|---|---|---|---|---|---|---|---|---|---|---|---|---|---|---|---|---|
| Polymer (wt%) | 1.25 | 1.25 | 1.25 | 1.25 | 1.25 | 1.25 | 1.25 | 1.25 | 1.25 | 1.25 | 1.25 | 1.25 | 1.25 | 1.25 | 1.25 | 1.25 |
| Peak viscosity (Pa•s) | 70.6 | 166.5 | 28.1 | 120.4 | 120.1 | 69.5 | 300.5 | 62.8 | 96.8 | 73.7 | 2.8 | 6.7 | 0.6 | 2.3 | 18.2 | 5.6 |
| Viscosity at 30 s⁻¹ (Pa•s) | 1.4 | 1.4 | 1.3 | 1.9 | 1.7 | 1.2 | 2.3 | 2.6 | 1.1 | 1.0 | 0.6 | 0.8 | 0.3 | 0.4 | 0.9 | 0.7 |

The instrument parameters included a lower temperature-controlled stainless-steel Peltier plate and an upper 40 millimeter stainless-steel 2 degree (°) cone. The conditioning step included conditioning at 25 °C for 10 seconds, pre-shearing at 21 inverse seconds (s⁻¹) for 10 seconds, and equilibrating for 300 seconds. The oscillation step occurred at 25 °C, at a constant strain of 1.0 percent, and used a logarithmic frequency sweep of 100 to 0.01 radians per second (rad/s) with 10 points per decade. The conditioning step was repeated and then a flow step was performed. The flow step included a logarithmic stress sweep at 0.1 to 400 Pascal (Pa) with 10 points per decade at a temperature of 25 °C, an equilibration time of 20 seconds, an averaging time of 30 seconds, and a scaled time average scale factor of 1.0.

The maximum viscosity value from the flow rheology curve was reported as "peak viscosity" in Table 2. The viscosity at 30 s⁻¹ was obtained by linear interpolation of the viscosities at the two shear rates closest to 30 s⁻¹ and also recorded in Table 2.

### Examples 5-7: Preparation of a personal care leave-on formulation (facial serum) containing wax beads

The following components were added to ajar containing 94.8 grams of deionized water while stirring: 3.0 g glycerol, 0.5 g of a preservative (propylene glycol, diazolidinyl urea, iodopropynyl butylcarbamate, GERMALL^{™} Plus, Ashland), and 1.6 g of an emulsion comprising 31.7 wt% of the polymer 1 of Example 1. The pH of the resulting composition was raised to 7.0 by the addition of triethanolamine and 0.1 g Jojoba wax beads (FLORAPEARLS* Jojoba STD Evergreen, Floratech) were added. The resulting composition of Example 5, which contained 0.5 wt% of a polymeric rheology modifier, had a Brookfield viscosity of 280 centipoise (cPs), was visually transparent (save for the beads), and was able to maintain the suspension of the beads for three weeks.

| Table 5 | | | |
|---|---|---|---|
| Example | 5 | 6 | 7 |
| Polymer 1 (wt%) | 0.5 | 0.75 | 1 |
| pH | 7.0 | 7.0 | 6.9 |
| Viscosity (cPs) | 280 | 1,000 | 2,160 |

Leave-on skin facial serum compositions of Examples 6 and 7 were prepared in accordance with Example 5 except that the amount of the polymer was changed. The results are shown in Table 5.

Table 5 shows that small changes in the amount of Polymer 1 resulted in significant changes in the resultant viscosity.

### Examples 8-10: Preparation of a leave-on color toner

The following components were added to ajar containing 94.8 grams of deionized water while stirring: 3.0 g glycerol, 0.5 g of a preservative (propylene glycol, diazolidinyl urea, iodopropynyl butylcarbamate, GERMALL^{™} Plus, Ashland), and 1.6 g of an emulsion comprising 31.7 wt% of the polymer of Example 1. The pH of the resulting dispersion was raised to 6.8 by the addition of triethanolamine. To the thickened formula was then added 0.1 g pigment particles (COLORONA^{™} SynCranberry synthetic fluorphlogopite, iron oxides, EMD Chemicals). The resulting composition of Example 8, which contained 0.5 wt% of a polymeric rheology modifier, had a Brookfield viscosity of 200 cPs, was homogeneous, and was able to maintain the suspension of the particles for three weeks.

Compositions of Examples 9 and 10 were prepared in accordance with Example 8 except that the amount of the polymer was changed. The results are shown in Table 6.

| Table 6 | | | |
|---|---|---|---|
| Example | 8 | 9 | 10 |
| Polymer 1 (wt%) | 0.5 | 0.75 | 1 |
| pH | 6.8 | 6.7 | 6.8 |
| Viscosity (cPs) | 200 | 400 | 2,240 |

Table 6 shows that small changes in the amount of Polymer 1 resulted in significant changes in the resultant viscosity.

The compositions, methods, and articles can alternatively comprise, consist of, or consist essentially of, any appropriate materials, steps, or components herein disclosed. The compositions, methods, and articles can additionally, or alternatively, be formulated so as to be devoid, or substantially free, of any materials (or species), steps, or components, that are otherwise not necessary to the achievement of the function or objectives of the compositions, methods, and articles.

As used herein, "a," "an," "the," and "at least one" do not denote a limitation of quantity, and are intended to cover both the singular and plural, unless the context clearly indicates otherwise. For example, "an element" has the same meaning as "at least one element," unless the context clearly indicates otherwise. The term "at least one of" means that the list is inclusive of each element individually, as well as combinations of two or more elements of the list, and combinations of at least one element of the list with like elements not named. Also, the term "combination" is inclusive of blends, mixtures, alloys, reaction products, and the like.

The term "or" means "and/or" unless clearly indicated otherwise by context. Reference throughout the specification to "an aspect", "an embodiment", "another embodiment", "some embodiments", and so forth, means that a particular element (e.g., feature, structure, step, or characteristic) described in connection with the embodiment is included in at least one embodiment described herein, and may or may not be present in other embodiments. In addition, it is to be understood that the described elements may be combined in any suitable manner in the various embodiments.

The endpoints of all ranges directed to the same component or property are inclusive of the endpoints, are independently combinable, and include all intermediate points and ranges. For example, ranges of "up to 25 wt%, or 5 to 20 wt%" is inclusive of the endpoints and all intermediate values of the ranges of "5 to 25 wt%," such as 10 to 23 wt%, etc. Moreover, stated upper and lower limits can be combined to form ranges, for example, "5 to 20 wt%, or 10 to 15 wt%" can be combined as the ranges "5 to 15 wt%," or " 10 to 20 wt%."

Unless defined otherwise, technical and scientific terms used herein have the same meaning as is commonly understood by one of skill in the art to which this disclosure belongs.

Compounds are described using standard nomenclature. For example, any position not substituted by any indicated group is understood to have its valency filled by a bond as indicated, or a hydrogen atom. A dash ("-") that is not between two letters or symbols is used to indicate a point of attachment for a substituent. For example, -CHO is attached through carbon of the carbonyl group. As used herein, the term "(meth)acryl" encompasses both acryl and methacryl groups.

## Claims

1. A composition comprising:
an alkali-swellable copolymer, wherein the alkali-swellable copolymer is derived from a mixture comprising:
15 to 50 wt% based on the total weight of the monomers in the mixture of an ethylenically unsaturated carboxylic acid;
30 to 70 wt% based on the total weight of the monomers in the mixture of an ethylenically unsaturated C₁₋₂ alkyl carboxylate ester;
15 to 40 wt% based on the total weight of the monomers in the mixture of a nonionic ethylenically unsaturated surfactant monomer of the Formula (A); 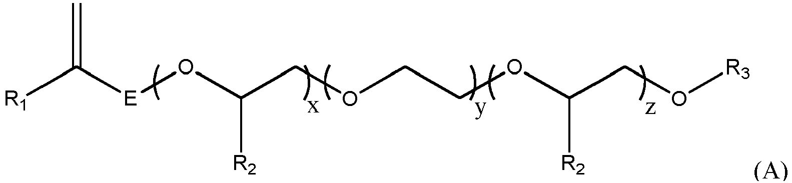
wherein R₁ is a methyl group or hydrogen, each R₂ independently is a methyl group or an ethyl group, R₃ is a linear C₈₋₁₄ alkyl group, x is an integer 0 to 3, y is an integer 10 to 40, z is an integer 0 to 5, and E is -(CH₂)-, -(CH₂CH₂)-, or -(C=O)-; and
less than or equal to 0.15 wt% based on the total weight of the monomers in the mixture of a multifunctional monomer containing greater than 1 vinyl group; and wherein the composition has a pH of greater than or equal to 6; and
wherein the composition optionally comprises a surfactant.

2. The composition of Claim 1, wherein the composition comprises 0.1 to 10 wt% of the alkali-swellable copolymer.

3. The composition of any one or more of the preceding claims, wherein the ethylenically unsaturated carboxylic acid comprises at least one of acrylic acid or methacrylic acid.

4. The composition of any one or more of the preceding claims, wherein the ethylenically unsaturated C₁₋₂ alkyl carboxylate ester comprises at least one of ethyl acrylate, ethyl methacrylate, methyl acrylate, or methyl methacrylate.

5. The composition of any one or more of the preceding claims, wherein the nonionic ethylenically unsaturated surfactant monomer comprises a methacrylate ester of a C₁₂₋₁₄ linear alcohol ethoxylate.

6. The composition of any one or more of the preceding claims, wherein the multifunctional monomer comprises at least one of trimethylolpropane triacrylate, trimethylolpropane diallyl ether, or diallyl phthalate.

7. The composition of any one or more of the preceding claims, wherein the alkali-swellable copolymer is free of a residue derived from a sulfonate monomer.

8. The composition of any one or more of the preceding claims, wherein the composition comprises greater than 0 to 50 wt% based on the total weight of the composition of the surfactant.

9. The composition of any one or more of the preceding claims, wherein the surfactant is present and comprises at least one of an anionic surfactant, a nonionic surfactant, or a zwitterionic surfactant.

10. The composition of Claim 9, wherein the surfactant comprises at least one anionic surfactant selected from the group consisting of sodium dodecylbenzenesulfonate, sodium laureth sulfate, sodium lauryl sulfate, sodium tridecyl ether sulfate, a dioctyl sulfosuccinate sodium salt, and a sodium salt of an alkylaryl polyether sulfonate.

11. The composition of Claim 9, wherein the surfactant comprises at least one nonionic surfactant selected from the group consisting of lauryl alcohol ethoxylate, an alkylaryl polyether alcohol, an alkylpolyglucoside, and an ethylene oxide-propylene oxide copolymer

12. The composition of Claim 9, wherein the surfactant comprises at least one zwitterionic surfactant selected from the group consisting of dimethyldodecylamine N-oxide and cocamidopropyl betaine.

13. The composition of any one or more of the preceding claims, wherein the composition is a cleaning composition or a personal care composition.

14. The composition of any one or more of the preceding claims, further comprising at least one of an insoluble component, an enzyme, a dispersant polymer, a conditioning polymer, a solvent, a hydrotropic electrolyte, an oxygen bleach, a bleach activator, an inorganic builder, an organic builder, a fatty acid, a fatty acid salt, a silicon fluid, an optical brightener, a dye transfer inhibitor, a dye, a fragrance, a salt, or a preservative.

15. A composition comprising:
0.1 to 10 wt% of an alkali-swellable copolymer based on the total weight of the composition, wherein the alkali-swellable copolymer is derived from a mixture comprising:
15 to 50 wt%, or 20 to 35 wt% based on the total weight of the monomers in the mixture of methacrylic acid and an optional acrylic acid, wherein the optional acrylic acid is present in an amount of less than or equal to 50 wt% based on the total weight of the methacrylic acid and the optional acrylic acid;
30 to 70 wt%, or 35 to 55 wt% based on the total weight of the monomers in the mixture of ethyl (meth)acrylate;
15 to 40 wt%, or 15 to 35 wt% based on the total weight of the monomers in the mixture of a nonionic ethylenically unsaturated surfactant monomer of the Formula (A); 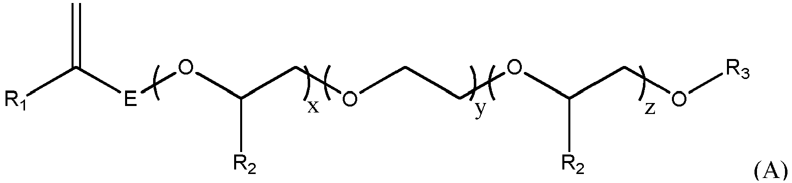
wherein R₁ is a methyl group or hydrogen, each R₂ independently is a methyl group or an ethyl group, R₃ is a linear C₁₂₋₁₄ alkyl group, x is an integer 0 to 3, y is an integer 10 to 40, z is an integer 0 to 5, and E is -(CH₂)-, -(CH₂CH₂)-, or -(C=O)-; and
0.01 to 0.15 wt% based on the total weight of the monomers in the mixture of a multifunctional monomer containing greater than 1 vinyl group; and wherein the composition has a pH of greater than or equal to 6; and
5 to 50 wt% of at least one of a nonionic surfactant or an anionic surfactant based on the total weight of the composition; and
wherein the alkali-swellable copolymer is the product of an aqueous emulsion polymerization.

## Patentansprüche

1. Zusammensetzung, umfassend:
ein alkaliquellbares Copolymer, wobei das alkaliquellbare Copolymer von einer Mischung stammt, umfassend:
zu 15 bis 50 Gew.-%, bezogen auf das Gesamtgewicht der Monomere in der Mischung, eine ethylenisch ungesättigte Carbonsäure;
zu 30 bis 70 Gew.-%, bezogen auf das Gesamtgewicht der Monomere in der Mischung, einen ethylenisch ungesättigten C₁₋₂-Alkylcarboxylatester;
zu 15 bis 40 Gew.-%, bezogen auf das Gesamtgewicht der Monomere in der Mischung, ein nichtionisches ethylenisch ungesättigtes Tensidmonomer der Formel (A); 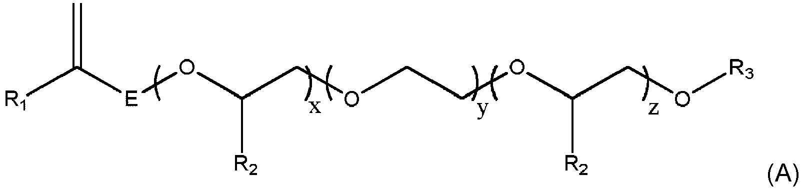
wobei R₁ eine Methylgruppe oder Wasserstoff ist, jedes R₂ unabhängig eine Methylgruppe oder eine Ethylgruppe ist, R₃ eine lineare C₈₋₁₄-Alkylgruppe ist, x eine ganze Zahl von 0 bis 3 ist, y eine ganze Zahl von 10 bis 40 ist, z eine ganze Zahl von 0 bis 5 ist und E -(CH₂)-, -(CH₂CH₂)- oder -(C=O)- ist; und
zu weniger als oder gleich 0,15 Gew.-%, bezogen auf das Gesamtgewicht der Monomere in der Mischung, ein multifunktionelles Monomer, das größer als 1 Vinylgruppe enthält; und wobei die Zusammensetzung einen pH-Wert aufweist, der größer als oder gleich 6 ist; und
wobei die Zusammensetzung optional ein Tensid umfasst.

2. Zusammensetzung nach Anspruch 1, wobei die Zusammensetzung zu 0,1 bis 10 Gew.-% das alkaliquellbare Copolymer umfasst.

3. Zusammensetzung nach einem oder mehreren der vorstehenden Ansprüche, wobei die ethylenisch ungesättigte Carbonsäure mindestens eine von Acrylsäure oder Methacrylsäure. umfasst.

4. Zusammensetzung nach einem oder mehreren der vorstehenden Ansprüche, wobei der ethylenisch ungesättigte C₁₋₂-Alkylcarboxylatester mindestens eines von Ethylacrylat, Ethylmethacrylat, Methylacrylat oder Methylmethacrylat umfasst.

5. Zusammensetzung nach einem oder mehreren der vorstehenden Ansprüche, wobei das nichtionische ethylenisch ungesättigte Tensidmonomer einen Methacrylatester eines linearen C₁₂₋₁₄-Alkoholethoxylats umfasst.

6. Zusammensetzung nach einem oder mehreren der vorstehenden Ansprüche, wobei das multifunktionelle Monomer mindestens eines von Trimethylolpropantriacrylat, Trimethylolpropandiallylether oder Diallylphthalat umfasst.

7. Zusammensetzung nach einem oder mehreren der vorstehenden Ansprüche, wobei das alkaliquellbare Copolymer frei von einem Rückstand ist, der von einem Sulfonatmonomer stammt.

8. Zusammensetzung nach einem oder mehreren der vorstehenden Ansprüche, wobei die Zusammensetzung zu größer als 0 bis 50 Gew.-%, bezogen auf das Gesamtgewicht der Zusammensetzung, das Tensid umfasst.

9. Zusammensetzung nach einem oder mehreren der vorstehenden Ansprüche, wobei das Tensid vorhanden ist und mindestens eines von einem anionischen Tensid, einem nichtionischen Tensid oder einem zwitterionischen Tensid umfasst.

10. Zusammensetzung nach Anspruch 9, wobei das Tensid mindestens ein anionisches Tensid umfasst, das aus der Gruppe ausgewählt ist, bestehend aus Natriumdodecylbenzolsulfonat, Natriumlaurethsulfat, Natriumlaurylsulfat, Natriumtridecylethersulfat, einem Natriumsalz von Dioctylsulfosuccinat und einem Natriumsalz eines Alkylarylpolyethersulfonats.

11. Zusammensetzung nach Anspruch 9, wobei das Tensid mindestens ein nichtionisches Tensid umfasst, das aus der Gruppe ausgewählt ist, bestehend aus Laurylalkoholethoxylat, einem Alkylarylpolyetheralkohol, einem Alkylpolyglucosid und einem Ethylenoxid-Propylenoxidcopolymer.

12. Zusammensetzung nach Anspruch 9, wobei das Tensid mindestens ein zwitterionisches Tensid umfasst, das aus der Gruppe ausgewählt ist, bestehend aus Dimethyldodecylamin-N-oxid und Cocamidopropylbetain.

13. Zusammensetzung nach einem oder mehreren der vorstehenden Ansprüche, wobei die Zusammensetzung eine Reinigungszusammensetzung oder eine Körperpflegezusammensetzung ist.

14. Zusammensetzung nach einem oder mehreren der vorstehenden Ansprüche, ferner umfassend mindestens eines von einer unlöslichen Komponente, einem Enzym, einem Dispergierpolymer, einem Konditionierungspolymer, einem Lösungsmittel, einem hydrotropen Elektrolyt, einem Sauerstoffbleichmittel, einem Bleichaktivator, einem anorganischen Builder, einem organischen Builder, einer Fettsäure, einem Fettsäuresalz, einem Silicon-Fluid, einem optischen Aufheller, einem Farbstoffübertragungsinhibitor, einem Farbstoff, einem Duftstoff, einem Salz oder einem Konservierungsmittel.

15. Zusammensetzung, umfassend:
zu 0,1 bis 10 Gew.-% ein alkaliquellbares Copolymer, bezogen auf das Gesamtgewicht der Zusammensetzung, wobei das alkaliquellbare Copolymer von einer Mischung stammt, umfassend:
zu 15 bis 50 Gew.-% oder 20 bis 35 Gew.-%, bezogen auf das Gesamtgewicht der Monomere in der Mischung, eine Methacrylsäure und eine optionale Acrylsäure, wobei die optionale Acrylsäure in einer Menge von weniger als oder gleich 50 Gew.-%, bezogen auf das Gesamtgewicht der Methacrylsäure und der optionalen Acrylsäure, vorhanden ist;
zu 30 bis 70 Gew.-% oder 35 bis 55 Gew.-%, bezogen auf das Gesamtgewicht der Monomere in der Mischung, Ethyl(meth)acrylat;
zu 15 bis 40 Gew.-% oder 15 bis 35 Gew.-%, bezogen auf das Gesamtgewicht der Monomere in der Mischung, ein nichtionisches ethylenisch ungesättigtes Tensidmonomer der Formel (A); 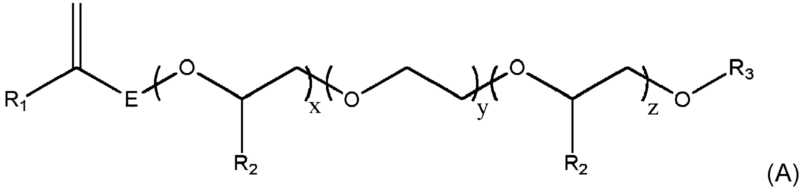
wobei R₁ eine Methylgruppe oder Wasserstoff ist, jedes R₂ unabhängig eine Methylgruppe oder eine Ethylgruppe ist, R₃ eine lineare C₁₂₋₁₄-Alkylgruppe ist, x eine ganze Zahl von 0 bis 3 ist, y eine ganze Zahl von 10 bis 40 ist, z eine ganze Zahl von 0 bis 5 ist und E -(CH₂)-, -(CH₂CH₂)- oder -(C=O)- ist; und
zu 0,01 bis 0,15 Gew.-%, bezogen auf das Gesamtgewicht der Monomere in der Mischung, ein multifunktionelles Monomer, das größer als 1 Vinylgruppe enthält; und wobei die Zusammensetzung einen pH-Wert aufweist, der größer als oder gleich 6 ist; und
zu 5 bis 50 Gew.-%, bezogen auf das Gesamtgewicht der Zusammensetzung, mindestens eines von einem nichtionischen Tensid oder einem anionischen Tensid; und
wobei das alkaliquellbare Copolymer das Produkt einer wässrigen Emulsionspolymerisation ist.

## Revendications

1. Composition comprenant :
un copolymère gonflable aux alcalins, dans lequel le copolymère gonflable aux alcalins est dérivé d'un mélange comprenant :
15 à 50 % en poids, sur la base du poids total des monomères dans le mélange, d'un acide carboxylique à insaturation éthylénique ;
30 à 70 % en poids, sur la base du poids total des monomères dans le mélange, d'un ester de carboxylate d'alkyle en C₁₋₂ à insaturation éthylénique ;
15 à 40 % en poids, sur la base du poids total des monomères dans le mélange, d'un monomère tensioactif non ionique à insaturation éthylénique de la Formule (A) ; 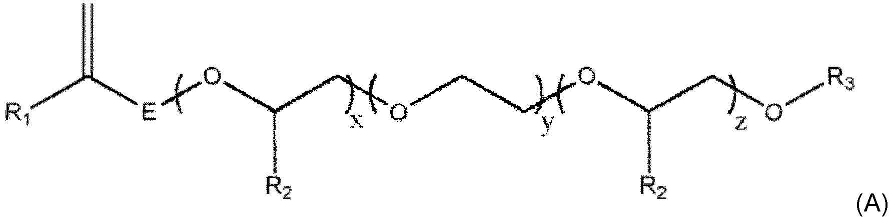
dans laquelle R₁ est un groupe méthyle ou hydrogène, chaque R₂ est indépendamment un groupe méthyle ou un groupe éthyle, R₃ est un groupe alkyle linéaire en C₈₋₁₄, x est un nombre entier de 0 à 3, y est un nombre entier de 10 à 40, z est un nombre entier de 0 à 5, et E est -(CH₂)-, -(CH₂CH₂)-, ou -(C=O)- ; et
0,15 % en poids ou moins, sur la base du poids total des monomères dans le mélange, d'un monomère multifonctionnel contenant plus de 1 groupe vinyle ; et dans laquelle la composition a un pH supérieur ou égal à 6 ; et
dans laquelle la composition comprend facultativement un agent tensioactif.

2. Composition selon la revendication 1, dans laquelle la composition comprend de 0,1 à 10 % en poids du copolymère gonflable aux alcalins.

3. Composition selon l'une ou plusieurs quelconques des revendications précédentes, dans laquelle l'acide carboxylique à insaturation éthylénique comprend au moins l'un parmi acide acrylique ou acide méthacrylique.

4. Composition selon l'une ou plusieurs quelconques des revendications précédentes, dans laquelle l'ester de carboxylate d'alkyle en C₁₋₂ à insaturation éthylénique comprend au moins l'un parmi acrylate d'éthyle, méthacrylate d'éthyle, acrylate de méthyle, ou méthacrylate de méthyle.

5. Composition selon l'une ou plusieurs quelconques des revendications précédentes, dans laquelle le monomère tensioactif non ionique à insaturation éthylénique comprend un ester de méthacrylate d'un éthoxylate d'alcool linéaire en C₁₂₋₁₄.

6. Composition selon l'une ou plusieurs quelconques des revendications précédentes, dans laquelle le monomère multifonctionnel comprend au moins l'un parmi triacrylate de triméthylolpropane, éther diallylique de triméthylolpropane, ou phtalate de diallyle.

7. Composition selon l'une ou plusieurs quelconques des revendications précédentes, dans laquelle le copolymère gonflable aux alcalins est exempt d'un résidu dérivé d'un monomère sulfonate.

8. Composition selon l'une ou plusieurs quelconques des revendications précédentes, dans laquelle la composition comprend plus de 0 à 50 % en poids, sur la base du poids total de la composition, de l'agent tensioactif.

9. Composition selon l'une ou plusieurs quelconques des revendications précédentes, dans laquelle l'agent tensioactif est présent et comprend au moins l'un parmi un agent tensioactif anionique, un agent tensioactif non ionique, ou un agent tensioactif zwitterionique.

10. Composition selon la revendication 9, dans laquelle l'agent tensioactif comprend au moins un tensioactif anionique choisi dans le groupe constitué de dodécylbenzènesulfonate de sodium, laureth sulfate de sodium, lauryl sulfate de sodium, tridécyl éther sulfate de sodium, un sel de sodium de sulfosuccinate de dioctyle, et un sel de sodium d'un alkylaryl polyéther sulfonate.

11. Composition selon la revendication 9, dans laquelle l'agent tensioactif comprend au moins un agent tensioactif non ionique choisi dans le groupe constitué d'éthoxylate d'alcool laurique, un alcool d'alkylaryl polyéther, un alkylpolyglucoside, et un copolymère oxyde d'éthylène-oxyde de propylène

12. Composition selon la revendication 9, dans laquelle l'agent tensioactif comprend au moins un agent tensioactif zwitterionique choisi dans le groupe constitué de N-oxyde de diméthyldodécylamine et de cocamidopropyl bétaïne.

13. Composition selon l'une ou plusieurs quelconques des revendications précédentes, dans laquelle la composition est une composition de nettoyage ou une composition de soins personnels.

14. Composition selon l'une ou plusieurs quelconques des revendications précédentes, comprenant en outre au moins l'un parmi un composant insoluble, une enzyme, un polymère dispersant, un polymère de conditionnement, un solvant, un électrolyte hydrotrope, un agent de blanchiment à l'oxygène, un activateur de blanchiment, un adjuvant inorganique, un adjuvant organique, un acide gras, un sel d'acide gras, un fluide siliconé, un azurant optique, un inhibiteur de transfert de colorant, un colorant, un parfum, un sel, ou un agent de conservation.

15. Composition comprenant :
0,1 à 10 % en poids d'un copolymère gonflable aux alcalins sur la base du poids total de la composition, dans laquelle le copolymère gonflable aux alcalins est dérivé d'un mélange comprenant :
15 à 50 % en poids, ou 20 à 35 % en poids, sur la base du poids total des monomères dans le mélange, d'acide méthacrylique et d'acide acrylique facultatif, dans laquelle l'acide acrylique facultatif est présent en une quantité inférieure ou égale à 50 % en poids sur la base du poids total de l'acide méthacrylique et de l'acide acrylique facultatif ;
30 à 70 % en poids, ou 35 à 55 % en poids, sur la base du poids total des monomères dans le mélange, de (méth)acrylate d'éthyle ;
15 à 40 % en poids, ou 15 à 35 % en poids, sur la base du poids total des monomères dans le mélange, d'un monomère tensioactif non ionique à insaturation éthylénique de la Formule (A) ; 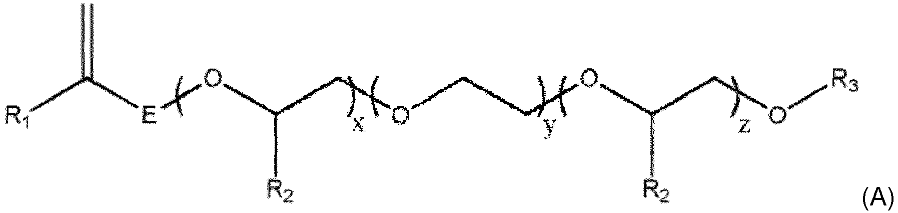
dans laquelle R₁ est un groupe méthyle ou hydrogène, chaque R₂ est indépendamment un groupe méthyle ou un groupe éthyle, R₃ est un groupe alkyle linéaire en C₁₂₋₁₄, x est un nombre entier de 0 à 3, y est un nombre entier de 10 à 40, z est un nombre entier de 0 à 5, et E est -(CH₂)-, -(CH₂CH₂)-, ou -(C=O)- ; et
0,01 à 0,15 % en poids, sur la base du poids total des monomères dans le mélange, d'un monomère multifonctionnel contenant plus de 1 groupe vinyle ; et dans laquelle la composition a un pH supérieur ou égal à 6 ; et
5 à 50 % en poids d'au moins l'un parmi un agent tensioactif non ionique ou un agent tensioactif anionique sur la base du poids total de la composition ; et
dans laquelle le copolymère gonflable aux alcalins est le produit d'une polymérisation en émulsion aqueuse.
